(19) **Europäisches Patentamt**
**European Patent Office**
**Office européen des brevets**

(11) **EP 4 195 210 A1**

(12) **EUROPEAN PATENT APPLICATION**

(43) Date of publication:
**14.06.2023 Bulletin 2023/24**

(21) Application number: **21306732.5**

(22) Date of filing: **09.12.2021**

(51) International Patent Classification (IPC):
**G16C 20/30** (2019.01)     **G16B 15/30** (2019.01)

(52) Cooperative Patent Classification (CPC):
**G16C 20/30; G16B 15/30**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Université de Lorraine**
  **54000 Nancy (FR)**
• **Centre national de la recherche scientifique**
  **75016 Paris (FR)**
• **INSTITUT NATIONAL DE LA SANTE ET DE LA RECHERCHE MEDICALE - INSERM**
  **75013 Paris (FR)**

(72) Inventors:
• **JELSCH, Christian**
  **54600 Villers les Nancy (FR)**
• **MITEVA, Maria**
  **92160 Antony (FR)**
• **GUILLOT, Benoit**
  **54200 Toul (FR)**

(74) Representative: **Gevers & Orès**
**Immeuble le Palatin 2**
**3 Cours du Triangle**
**CS 80165**
**92939 Paris La Défense Cedex (FR)**

(54) **METHOD FOR EVALUATING THE STABILITY OF THE STRUCTURE OF A MOLECULE-ENVIRONMENT COMPLEX**

(57)     The present invention concerns a computer based method for evaluating the stability of at least one structure of at least one complex constituted of a molecule and its environment.

**Description**

The present invention concerns a computer based method for evaluating the stability of at least one structure of at least one complex constituted of a molecule and its environment.

The lifecycle for the discovery of a new drug typically spans ten to fifteen years and about 75% of the overall research and development expenditures are attributed to failures.

Thus, the study of protein-ligand interactions has been an area of active research with widespread implications, especially its potential impact on rational drug discovery and design. In particular, the ability to score protein-ligand interactions and to predict binding affinity could have a major impact on accelerating the discovery of low molecule weight ligands that potently and specifically bind to a target. Additionally, the ability to rank analog series could facilitate the identification of alternative compounds in the presence of constraints imposed by pharmacokinetics and toxicity, thus reducing the likelihood of costly late stage failures.

Generally speaking, a crystal is a periodic arrangement of molecules arranged in space. The crystal retained in nature will be the one that has a minimum free energy. The same applies to protein-ligand interactions, wherein the conformation of the protein and the ligand retained in nature will be among the ones of lower free energy.

Computational drug design methods and in particular method for scoring protein-ligand interactions and binding affinity predictions, based on a description of the energetic components of binding, has thus proven to be a major challenge.

Virtual screening of ligands in a chemical library of thousands or millions of molecules makes it possible to find compounds that have a high probability of having an affinity for a protein target.

Many tools and software dedicated to docking and crystal packing prediction exist. The generation of docking poses or crystal packings uses various sophisticated algorithms and is not the subject of the present invention.

However, these methods remain uncertain and a docking pose always requires experimental confirmation by radiocrystallography or NMR for example. The results are indeed not certain when it comes to determining the correct pose (positioning of the ligand) or estimating a ligand's affinity (association constant Km).

Various scoring functions already exist to evaluate docking poses and to estimate the affinity or energy constant.

Docking and scoring are interrelated in computational drug design and the success of one depends on the other.

Docking of compounds into a receptor binding site and then scoring the docked "pose" is a commonly used methodology for identifying lead compounds. A critical part of drug discovery is in lead optimization, and it is here that the accuracy of scoring functions is more important than the speed of the scoring. Docking algorithms have been reasonably successful in predicting some binding modes. However, scoring of the poses in order to predict the correct pose and the binding free energy has proven much more challenging.

Empirical functions calibrate for example the free energy of protein/ligand interaction by making combinations such as:

$$E(total) \; = \; a \; E(electrostatic) \; + \; b \; E(polarization) \; + \; c \; E(van.der.Waals) \; + \; d \; E(solvation) \; + \; e \; E(entropy)$$

The docking pose with the lowest energy has the highest probability of being the correct one. In the final phase, molecular dynamics simulations can be performed to evaluate docking poses by seeing if the ligand remains docked in the binding site over a long period of time. However, these simulations are extremely costly in computation time.

In the case of crystal packings, the criterion for determining the most probable crystal is to calculate the energy by means of time-consuming and expensive quantum calculations.

Electrostatic forces generally account for a significant proportion of the interaction energy between molecules and may be responsible for the selection of a pairing mode.

The analysis of the electrostatic complementarity between a molecule and its neighbors in its crystal environment can be visualized by (Vint,Vext)-type scatter plots, wherein Vint is the electrostatic potential generated by the molecule, and Vext is the electrostatic potential generated by the neighboring molecules. The (Vint,Vext) values are typically obtained on a surface delimiting the molecule and its environment, as for example the Hirshfeld surface.

The (Vint,Vext) graphs reveal in general a certain anti-correlation between the inner and outer potentials, but the result is not at all clear-cut.

Accordingly, it is an object of the present invention to provide an easy-access method enabling the evaluation of the stability of a structure, with no need of time-consuming and expensive calculations, in particular quantum calculations.

Inventors have for the first time demonstrated that a method comprising a step of determination of the interior and exterior electrostatic energy densities of a complex enables the evaluation of the stability of said complex.

The method of the invention can for example be seen as a filter that may be applied as an intermediate step on the various techniques and software used in the field of molecular recognition prediction, for instance:

- docking a ligand on a protein;
- virtual protein/ligand screening in a chemical library;
- engineering a protein to make it recognise a given ligand;
- protein/protein docking;
- crystal structure prediction.

This filter may for example be very useful in the big data context to improve the selection process of a virtual screening.

The method of the invention is able to give a number to score docking poses but also provides a two-dimensional diagram to analyze the interactions between molecules. This 2D diagram can thus be used to map a ligand in a protein and give leads to increase (or decrease) its affinity.

In addition, the selectivity of a ligand towards a therapeutic target can also be adjusted thanks to a method of the invention by comparing the diagrams obtained on several proteins of the same family.

Such a filter can also be used to highlight non-optimal docking positions and eliminate false positives. The efficiency, the "enrichment rate" of a virtual screening would thus be increased as well.

Thus, in one aspect, the present invention relates to a computer based method for evaluating the stability of at least one structure of at least one complex constituted of a molecule and its environment, comprising the following steps of:

(i) Receiving at least one structure of at least one complex constituted of a molecule and its environment;

(ii) Defining from the structure received in step (i) a surface delimiting said molecule from said environment;

(iii) Determining for each point of a plurality of points of the surface defined in step (ii) the electrostatic energy density $E\_elec\_int$ computed as the product of the electrostatic potential (ESP) generated by said molecule and the electron density generated by said environment, and the electrostatic energy density $E\_elec\_ext$ computed as the product of the electrostatic potential generated by said environment and the electron density generated by said molecule, or the descriptor $F\_int$ computed as the product of the electrostatic potential (ESP) generated by said molecule and (electron density)$^p$, with p between 0 and 1, generated by said environment, and the descriptor $F\_ext$ computed as the product of the electrostatic potential generated by said environment and (electron density)$^p$, with p between 0 and 1, generated by said molecule.

In particular, the present invention relates to a computer based method for evaluating the stability of at least one structure of at least one complex constituted of a molecule and its environment, comprising the following steps of:

(i) Receiving at least one structure of at least one complex constituted of a molecule and its environment;

(ii) Defining from the structure received in step (i) a surface delimiting said molecule from said environment;

(iii) Determining for each point of a plurality of points of the surface defined in step (ii) the electrostatic energy density $E\_elec\_int$ computed as the product of the electrostatic potential (ESP) generated by said molecule and the electron density generated by said environment, and the electrostatic energy density $E\_elec\_ext$ computed as the product of the electrostatic potential generated by said environment and the electron density generated by said molecule.

The electrostatic energy density, named $E\_elec$, can also be referred as EED.

In a particular embodiment, the invention concerns the computer based method as defined below, comprising the following steps of:

(i) Receiving at least one structure of at least one complex constituted of a molecule and its environment;

(ii) Defining from the structure received in step (i) a surface delimiting said molecule from said environment;

(iii) Determining for each point of a plurality of points of the surface defined in step (ii) the electrostatic energy density $E\_elec\_int$ computed as the product of the electrostatic potential (ESP) generated by said molecule and the electron density generated by said environment, and the electrostatic energy density $E\_elec\_ext$ computed as the product of the electrostatic potential generated by said environment and the electron density generated by said molecule, or the descriptor $F\_int$ computed as the product of the electrostatic potential (ESP) generated by said molecule and (electron density)$^p$, with p between 0 and 1, generated by said environment, and the descriptor $F\_ext$ computed as the product of the electrostatic potential generated by said environment and (electron density)$^p$, with p between 0 and 1, generated by said molecule;

(iv) Estimating for the at least one structure of the at least one complex the complementarity of said molecule with said environment by describing the plurality of points according to their (E_elec_int, E_elec_ext) or (F_int, F_ext) couple of values as determined in step (iii).

In a particular embodiment, the molecule is an organic compound, an organometallic compound, or an inorganic compound, having a molecular weight of less than 5000 or 10000 Da, in particular:

- an organic compound, an organometallic compound, or an inorganic compound, having a molecular weight of less than 900 Da, or
- a peptide or a foldamer, in particular having a molecular weight between 900 and 5000 or 10000 Da.

In a particular embodiment, the molecule is a biopolymer, in particular a protein or a nucleic acid.
By "biopolymers" is in particular meant natural polymers produced by the cells of living organisms. Biopolymers generally consist of monomeric units that are covalently bonded to form larger molecules. There are three main classes of biopolymers, classified according to the monomers used and the structure of the biopolymer formed: polynucleotides, polypeptides, and polysaccharides. Polynucleotides, such as RNA and DNA, are long polymers composed of nucleotide monomers. Polypeptides and proteins are polymers of amino acids, they include enzymes and some major examples are collagen, actin, and fibrin. Polysaccharides are linear or branched polymeric carbohydrates and examples include starch, cellulose and alginate. Other examples of biopolymers include natural rubbers (polymers of isoprene), suberin and lignin (complex polyphenolic polymers), cutin and cutan (complex polymers of long-chain fatty acids) and melanin.
In a particular embodiment, the environment is constituted of or comprises a biopolymer, in particular a protein or a nucleic acid.
In a particular embodiment, the environment is constituted of or comprises a plurality of said molecule, said molecule being in particular an organic compound, an organometallic compound, or an inorganic compound, having a molecular weight of less than 5000 or 10000 Da.
In a particular embodiment, the environment is constituted of said molecule, said molecule being in particular an organic compound, an organometallic compound, or an inorganic compound, having a molecular weight of less than 5000 or 10000 Da.
The complex is then a homodimer or a hetereodimer.
In a particular embodiment, the complex is a ligand-biopolymer complex, in particular a ligand-protein complex, wherein the ligand is more particularly an organic compound having a molecular weight lower than 5000 or 10000 Da, even more particularly an organic compound having a molecular weight lower than 900 Da or an organic compound having a molecular weight between 900 and 5000 or 10000 Da.
In a particular embodiment, the complex is a crystalline structure, in particular a crystal packing, in particular constituted of a molecule and of its environment of molecules of the same nature, said molecule being more particularly an organic compound, an organometallic compound, or an inorganic compound, having a molecular weight of less than 5000 or 10000 Da.
In a particular embodiment, the complex is a metal organic framework (MOF), in particular constituted of a small molecule and of its MOF environment, said molecule being more particularly an organic compound, an organometallic compound, or an inorganic compound, having a molecular weight of less than 5000 or 10000 Da.
In a particular embodiment, the complex is made of an inorganic porous matrix and a small molecule, the inorganic porous matrix being in particular a zeolite and said small molecule being more particularly an organic compound, an organometallic compound, or an inorganic compound, having a molecular weight of less than 10 000 Da.
In a particular embodiment, the complex is an assembly of two biopolymers, said biopolymers being in particular proteins or nucleic acids.
In a particular embodiment, the surface defined in step (ii) is a Hirshfeld surface around the molecule, a shell of space around said Hirshfeld surface, a Hirshfeld surface shifted toward the interior, or a Hirshfeld surface shifted towards the environment.
By a "Hirshfeld surface" is in particular meant a surface around the molecule obtained by the equation $H(\mathbf{r}) = \frac{1}{2}$ where the function H is defined at position r by H = rho_int / ( rho_int + rho_ext ), rho_int and rho_ext being an electron density of the molecule and of its environment respectively, in particular the total electron density or the spherical electron density. By definition of the Hirshfeld surface, the electron density of the molecule thus equals the electron density of its environment on said Hirshfeld surface.
Alternatively, the surface can be an equidistance surface between the inner molecule and its environment. By an "equidistance surface" is in particular meant a surface around the molecule obtained by the equation $H(\mathbf{r}) = \frac{1}{2}$ where the function $H$ is defined at position r by $H$ = distance_int / ( distance_int + distance_ext ), distance_int and distance_ext being the shortest distance to the atom nuclei of the molecule and of its environment, respectively. On this equidistance surface, the electron density is taken as the sum or as the average of electron densities of the molecule and of its environment.

Alternatively, the surface can be an equidistance over van-der-Waals radius surface between the inner molecule and its environment. By an "equidistance over van-der-Waals radius surface" is in particular meant a surface around the molecule obtained by the equation $H(\mathbf{r})$ = ½ where the function H is defined at position r by H = DW_int / ( DW_int + DW_ext), DW being the smallest [distance over atomic van der Waals radius] ratio. DW_int and DW_ext are smallest distance/rvdWaals ratios to the atom nuclei of the molecule and of its environment, respectively. On this equi-distance/rvd-Waals surface, the electron density is taken as the sum or as the average of electron densities of the molecule and of its environment.

By a "shell of space around the surface" is in particular meant the region of space where $a$ < H(r) < $b$ where $a$ and $b$ are two real numbers between 0 and 1.

By a "surface shifted toward the interior" is in particular meant a surface H(r) = a where ½ <$a$<1.

By a "surface shifted toward the environment" is in particular meant a surface H(r) = a where 0 < $a$ < ½.

The Hirshfeld surface or a Hirshfeld shell is an appropriate region around a molecule to compute the electron density, the ESP and the resulting EES. The electron density generated by the molecule is indeed equal to the electron density generated by the environment, on the Hirshfeld surface. Therefore, the interior and exterior EEDs computed on a point of the Hirshfeld surface are obtained by multiplying the interior and exterior ESP by the same electron density value.

On the other hand, a scatterplot on an isosurface of electron density is similar to a plot of interior and exterior ESP, which is less informative on the electrostatic complementarity.

In another particular embodiment, the electrostatic complementarity can be evaluated on other surfaces, in respect to step (ii), such as the interior molecule van der Waals surface of the said inner molecule to yield EED_int and EED_ext scatterplots as defined in step (iii).

In a particular embodiment, the electrostatic potential is:

- The total electrostatic potential (ESP_tot) generated by the molecule;
- The deformation electrostatic potential (ESP_def), corresponding to the difference between the total electrostatic potential and the theoretical electrostatic potential generated by atoms all with zero charge and spherical electron density;
- The spherical electrostatic potential (ESP_sph), corresponding to the total electrostatic potential without any multipolar component;
- The spherical deformation electrostatic potential (ESP_def_sph), corresponding to the difference between the spherical electrostatic potential and the theoretical electrostatic potential generated by atoms all with zero charge and spherical electron density;
- The punctual electrostatic potential (ESP_punctual) computed from formal charges $\delta q$ placed at the atomic nuclei;
- The "non-nucleus" electrostatic potential (ESP_nonuc), corresponding to a potential computed from a modified charge density wherein the nucleus point charge of all atoms is replaced by a positive spherical charge density rho_proton(r) centered on the nucleus which has the same distribution as a function distance r to the nucleus as rho_core(r), the atomic electron density of core electrons; or
- The "non-nucleus" spherical electrostatic potential (ESP_nonuc_sph), corresponding to a potential computed from a modified spherical charge density wherein the nucleus point charge of all atoms is replaced by a positive spherical charge density rho_proton(r) centered on the nucleus which has the same distribution as a function distance r to the nucleus as rho_core(r), the atomic electron density of core electrons.

The deformation electrostatic potential, ESP_def can thus be obtained as follows: ESP_def = ESP_tot - ESP_sph,neu, wherein ESP_tot is the total electrostatic potential and ESP_sph,neu is the theoretical electrostatic potential generated by atoms all with zero charge and spherical electron density.

Regarding the "non-nucleus" electrostatic potential, the atomic rho_proton(r) density is in particular multiplied by a normalization coefficient $Z/N$core to ensure that the integration over space yields Z, the atomic number of the chemical species. Ncore is in particular the number of electrons in the atomic core shell. For an atom, the "non-nucleus" electrostatic potential ESP_nonuc is thus as follows: ESP_nonuc = ESP_tot - ESP_nucl - ESP_core * Z/Ncore.

The spherical electrostatic potential and the punctual electrostatic potential may for example be used when the computed electrostatic potential is related to a protein.

A potential being the total potential or the nonuc potential minus its average value on the Hirshfeld molecular surface can also be used, for example when the molecules interacting are charged, such as for a salt.

By "charge density of a molecule" is in particular meant the summation over its constituting atoms of the nucleus point charge and the atomic electron densities. The electrostatic potential is more particularly computed from the charge density using the Coulomb law.

The charge assigned to all atoms of the interacting molecules can for example be derived from the ELMAM2 library describing the electron density of common chemical groups (Domagala, S. et al. 2012, Acta Crystallographica Section A: Foundations of Crystallography, 68(3), 337-351).

In a particular embodiment, the electron density is:

- The total electron density (rho_tot);
- The spherical neutral electron density (rho_sph_neu), corresponding in particular to an approximation of the total electron density using a superposition of atoms with all zero charge and spherical electron density;
- The spherical electron density (rho_sph), corresponding in particular to an approximation of the total electron density using a superposition of atoms with spherical electron density;
- The deformation electron density (rho_def), corresponding in particular to the difference between the total electron density and the theoretical electron density generated by atoms all with zero charge and spherical electron density; or
- The spherical deformation electron density (rho_def_sph), corresponding in particular to the difference between the spherical electron density and the theoretical electron density generated by atoms all with zero charge and spherical electron density; or
- The punctual charge density (rho_punctual), corresponding to atomic partial charges placed at the nuclei
- The "non-nucleus" charge density (rho_nonuc) corresponding to a modified charge density where the nucleus point charge of all atoms is replaced by a positive spherical charge density rho_proton(r) centered on the nucleus which has the same distribution as a function of distance r to the nucleus as rho_core(r), the atomic electron density of core electrons.
- The "non-nucleus" spherical charge density (rho_nonuc_sph) corresponding to a modified spherical charge density where the nucleus point charge of all atoms is replaced by a positive spherical charge density rho_proton(r) centered on the nucleus which has the same distribution as a function of distance r to the nucleus as rho_core(r), the atomic electron density of core electrons.

The total electron density can be computed as a summation over all the atoms of the molecules, using a multipolar atom model.

The deformation electron density rho_def can thus be obtained as follows: rho_def = rho_tot - rho_sph_neu, wherein rho_tot is the total electron density and rho_sph_neu is the theoretical electron density generated by atoms all with zero charge and spherical electron density. The spherical deformation electron density rho_def_sph can thus be obtained as follows: rho_def = rho_sph - rho_sph_neu, wherein rho_sph is the spherical electron density and rho_sph_neu is the theoretical electron density generated by atoms all with zero charge and spherical electron density.

Regarding the "non-nucleus" charge density, the atomic rho_proton(r) density is in particular multiplied by a normalization coefficient to ensure that the integration over space yields Z, the atomic number of the chemical species. The "non-nucleus" charge density Rho_nonuc is thus as follows: Rho_nonuc = Rho_tot - Rho_nucl - Rho_core * Z/Ncore.

In a particular embodiment, the electrostatic energy density corresponds to E_elec_tot, the product of the total electrostatic potential and the electron density, said electron density being in particular the total electron density, the spherical electron density or the spherical neutral electron density.

In a particular embodiment, the electrostatic energy density corresponds to E_elec_def, the product of the deformation electrostatic potential and the electron density, said electron density being in particular the total electron density, the spherical electron density or the spherical neutral electron density.

In a particular embodiment, the electrostatic energy density corresponds to E_elec_def_sph, the product of the spherical deformation electrostatic potential and the electron density, said electron density being in particular the total electron density, the spherical electron density or the spherical neutral electron density.

In a particular embodiment, the electrostatic energy density corresponds to E_elec_punctual, the product of the punctual electrostatic potential and the electron density, said electron density being in particular the total electron density, the spherical electron density or the spherical neutral electron density.

E_elec_punctual is well balanced positively and negatively and may be an alternative to E_elec_nonuc as described below.

In a particular embodiment, the electrostatic energy density corresponds to E_elec_nonuc, the product of the "non-nucleus" electrostatic potential and the electron density, said electron density being in particular the total electron density or the spherical neutral electron density.

In a particular embodiment, the electrostatic energy density corresponds to E_elec_nonuc_sph, the product of the "non-nucleus" spherical electrostatic potential and the electron density, said electron density being in particular the total electron density, the spherical electron density or the spherical neutral electron density.

In a particular embodiment, the electrostatic energy density corresponds to E_elec_punctual, the product of the punctual electrostatic potential and the electron density, said electron density being in particular the total electron density, the spherical electron density or the spherical neutral electron density.

Punctual charges may be transferred for example from the AMBER dictionary in the case of a protein. The punctual ESP may be directly computed from these charges. Alternatively, a spherical ESP, a spherical deformation ESP or a spherical_non-nucleus ESP may be derived from these punctual charges.

In a particular embodiment, the electrostatic energy density corresponds to a linear combination of E_elec_tot, and E_elec_def, as defined above.

In a particular embodiment, the electrostatic energy density corresponds to a linear combination of E_elec_nonuc, and E_elec_def, as defined above.

In a particular embodiment, the electrostatic energy density corresponds to a linear combination of E_elec_nonuc_sph, and E_elec_def_sph, as defined above.

In a particular embodiment, the plurality of points are represented in step (iv) in a 2D diagram according to their (E_elec_int, E_elec_ext) coordinates.

For all the (E_elec_int, E_elec_ext) coordinates defined in the present specification, E_elec_int and E_elec_ext may be independently computed as defined below, for example independently chosen from E_elec_tot, E_elec_def, E_elec_sph, E_elec_punctual, E_elec_nonuc, E_elec_nonuc_sph, E_elec_def_sph, and their linear combinations.

For all the (E_elec_int, E_elec_ext) coordinates defined in the present specification, E_elec_int and E_elec_ext may be independently computed as the product of the electrostatic potential chosen from ESP_tot, ESP_def, ESP_sph, ESP_punctual, ESP_nonuc, ESP_nonuc_sph, ESP_def_sph, and their linear combinations, and the electron density chosen from rho_tot, rho_sph, rho_sph_neu, rho_nonuc, rho_nonuc_sph, and their linear combinations.

In a particular embodiment, the plurality of points are represented in step (iv) in a 2D diagram according to their (E_elec_int, E_elec_ext) coordinates wherein each of the two electrostatic energy densities, not necessarily the same, are chosen from E_elec_tot, E_elec_sph, E_elec_def, E_elec_def_sph, E_elec_nonuc, E_elec_nonuc_sph, E_elec_punctual, and their linear combinations.

In a particular embodiment, the plurality of points are represented in step (iv) in a 2D diagram according to their (E_elec_int, E_elec_ext) coordinates wherein each of the two electrostatic energy densities are a linear combination, not necessarily the same, of E_elec_tot, E_elec_sph, E_elec_def, E_elec_def_sph, E_elec_nonuc, E_elec_nonuc_sph and/or E_elec_punctual.

In a particular embodiment, the plurality of points are represented in step (iv) in a 2D diagram according to their (E_elec_tot_int, E_elec_tot_ext) coordinates, wherein E_elec_tot corresponds to the product of the total electrostatic potential and the electron density, said electron density being in particular the total electron density, the spherical electron density or the spherical neutral electron density.

In a particular embodiment, the plurality of points are represented in step (iv) in a 2D diagram according to their (E_elec_sph_int, E_elec_sph_ext) coordinates, wherein E_elec_sph corresponds to the product of the spherical electrostatic potential and the electron density, said electron density being in particular the total electron density, the spherical electron density or the spherical neutral electron density.

In a particular embodiment, the plurality of points are represented in step (iv) in a 2D diagram according to their (E_elec_nonuc_int, E_elec_nonuc_ext) coordinates, wherein E_elec_nonuc corresponds to the product of the "non-nucleus" electrostatic potential and the electron density, said electron density being in particular the total electron density, the spherical electron density or the spherical neutral electron density.

In a particular embodiment, the plurality of points are represented in step (iv) in a 2D diagram according to their (E_elec_nonuc_sph_int, E_elec_nonuc_sph_ext) coordinates, wherein E_elec_nonuc_sph corresponds to the product of the "non-nucleus" spherical electrostatic potential and the electron density, said electron density being in particular the total electron density, the spherical electron density or the spherical neutral electron density.

In a particular embodiment, the plurality of points are represented in step (iv) in a 2D diagram according to their (E_elec_punctual_int, E_elec_punctual_ext) coordinates, wherein E_elec_punctual corresponds to the product of the punctual electrostatic potential and the electron density, said electron density being in particular the total electron density, the spherical electron density or the spherical neutral electron density.

The "nonuc" electrostatic energy density may enable to attenuate non-linear effects for the total EED observed for strong interactions.

In a particular embodiment, the plurality of points are represented in step (iv) in a 2D diagram according to their (E_elec_def_int, E_elec_def_ext) coordinates, wherein E_elec_def corresponds to the product of the deformation electrostatic potential and the electron density, said electron density being in particular the total electron density, the spherical electron density or the spherical neutral electron density.

In particular, the "deformation" electrostatic energy density eliminates non-linear effects for the total EED observed for strong interactions, two different slopes being however often observed in the (+,-) and (-,+) quadrants.

In a particular embodiment, the plurality of points are represented in step (iv) in a 2D diagram according to their (E_elec_int, E_elec_ext) coordinates, wherein E_elec corresponds to a linear combination of E_elec_tot, and E_elec_def, as defined above.

In a particular embodiment, the plurality of points are represented in step (iv) in a 2D diagram according to their (E_elec_int, E_elec_ext) coordinates, wherein E_elec corresponds to a linear combination of E_elec_nonuc, and E_elec_def, as defined above.

Linear Combinations of (E_nonuc, E_def) or of (E_tot,E_def) may enable to observe linear segments instead of circle segments (crescent) in the case of E_tot, for a strong interaction in the (E_int,E_ext) cloud of points.

Linear Combinations of (E_nonuc, E_def) or of (E_tot, E_def) may enable to obtain an (E_int, E_ext) cloud of points which is close to the y = -x line when the interacting molecules have a good electrostatic complementarity.

Linear Combinations of (E_nonuc_sph, E_def_sph) or of (E_nonuc_sph, E_def) or of (E_nonuc, E_def_sph) or of (E_sph, E_def_sph) may enable to obtain an (E_int, E_ext) cloud of points which is close to the y = -x line when the interacting molecules have a good electrostatic complementarity.

In a linearized (E_int,E_ext) graph, the correlation coefficient of internal and external EED values may then be an appropriate descriptor to give a score to a set of molecular interactions.

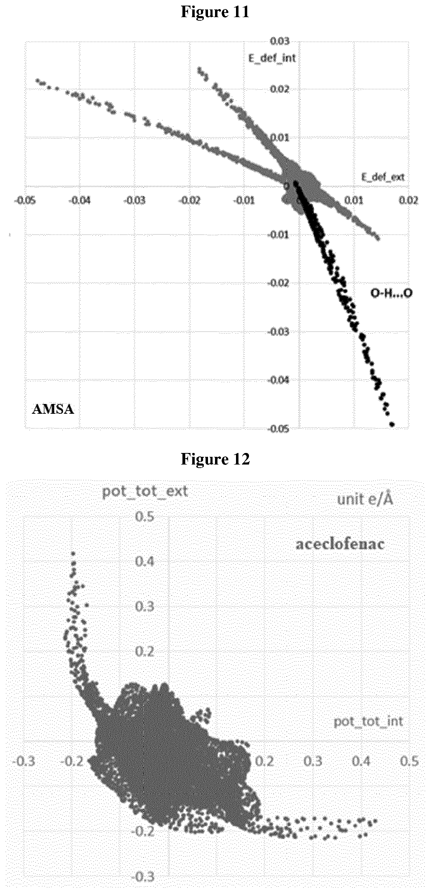

A strong interatomic interaction, either favorable or unfavorable, can be associated to a straight segment in linearized (EED_int,EED_ext) graph (Figure 11).

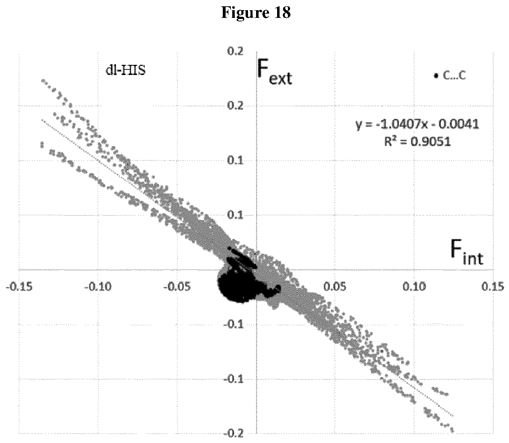

Indeed, for each point (E_elec_int,E_elec_ext), the two interacting atoms can be retrieved and can be highlighted in the graph. For example, in the case of a DL-histidine crystal, each branch corresponds to a hydrogen bond, and the atoms that correspond to a segment have been identified. In addition, the region of weak negative (Fint,Fext) values, as defined below, correspond to C...C interactions, which correspond to stacking interactions between two imidazole cycles (Figure 18).

In particular, the 2D (E_elec_int, E_elec_ext) diagram reveals in the (E_elec_int>0, E_elec_ext<0) and/or (E_elec_int<0, E_elec_ext>0) quadrants the favorable interactions between the molecule and its environment.

The 2D (E_elec_int, E_elec_ext) diagram may also reveal, around the origin of coordinates, in the regions of small E_elec_int and E_elec_ext values, the intermolecular interactions between atoms with a small charge, in particular the hydrophobic contacts, which are considered as favorable to the stability of the complex.

It is noted that the 2D scatterplot of (Eelec_int; E_elec_ext) electrostatic energy density of the invention is much more informative qualitatively and quantitative on the electrostatic complementarity of the two molecular entities forming the complex than the 2D scatterplot of (ESP_int, ESP_ext) electrostatic potential as the long distance, less important contact zones, are shifted towards the origin (0.,0.) of the 2D diagram.

In a particular embodiment, the analysis step (iv) can be followed by a step (v) of optimizing the interactions between the molecule and its environment by identifying unfavorable interactions, in particular in the (E_elec_int>0, E_elec_ext>0) and/or (E_elec_int<0, E_elec_ext<0) quadrants.

In a particular embodiment, the describing of step (iv) is performed by fitting the simple linear regression line of said plurality of points, or of part of said plurality of points, and/or by computing the linear correlation coefficient R of said plurality of points, or of part of said plurality of points.

R can be obtained by summation over the plurality of points i, or over part of said plurality of points : $R = \Sigma_i$ [ E_elec_int(i) * E_elec_ext(i) ] / [ $[\Sigma_i$ Elec-int(i)$]^2$ * $[\Sigma_i$ Elec-ext(i)$]^2$ $]^{\frac{1}{2}}$.

The slope of the simple linear regression line (E_elec_ext = a * E_elec_int + b) of said plurality of points can in particular indicate the quality of the complex stabilization. A slope close to -1. and a correlation coefficient R close to -1 may indicate that all interactions are very favorable. A slope close to +1. and a correlation coefficient R close to +1 may indicate that all interactions are unfavorable.

In a particular embodiment, the describing of step (iv) is performed by (a) fitting the simple linear regression line of said plurality of points, or of part of said plurality of points, and (b) displaying a representation of said surface indicating for the plurality of points or part of it the deviation D from the regression line.

Said deviation D can be computed as $D_1$ or $D_2$, as follows:

$$D_1 = \Sigma_i \, abs(E_i - E_{i,fit}) \, / \, \Sigma_i \, abs(E_i) \; ; \text{ or } D_2 = [\, \Sigma_i \, (E_i - E_{i,fit})^2 \, / \, \Sigma_i \, E_i^2 \,]^{\frac{1}{2}} \; ;$$

where $E_i$ is the external electrostatic potential density and $E_{i,fit}$ is the external electrostatic potential density fitted as a function of the internal one.

Alternatively, $E_i$ can also be the internal electrostatic potential density fitted as a function of the external one.

In a particular embodiment, the analysis step (iv) is followed by a step (v) of optimizing the interactions between the molecule and its environment by identifying the region(s) where the deviation from the regression line are the most important.

In a particular embodiment, the invention concerns a computer based method as defined above, for evaluating and comparing the stability of a plurality of structures, by computing, for each structure, the linear correlation coefficient R of said plurality of points, or of part of said plurality of points.

In a particular embodiment, the plurality of structures corresponds to different structures or poses of a same molecule-environment couple.

In a particular embodiment, the plurality of structures corresponds to the molecular structures or poses of different molecule-environment complexes, the molecule or the environment being the same for all the complexes.

In a particular embodiment, the invention concerns a computer based method as defined above, for evaluating and comparing the stability of at least one structure of a plurality of complexes constituted of a molecule and its environment.

In a more particular embodiment, the plurality of complexes corresponds to real or predicted crystal packings, the molecule being the same for all the complexes.

In a more particular embodiment, the invention concerns a computer based method as defined above, the plurality of complexes corresponds to real or predicted co-crystals or crystals of salts, the molecule being the same for all the complexes.

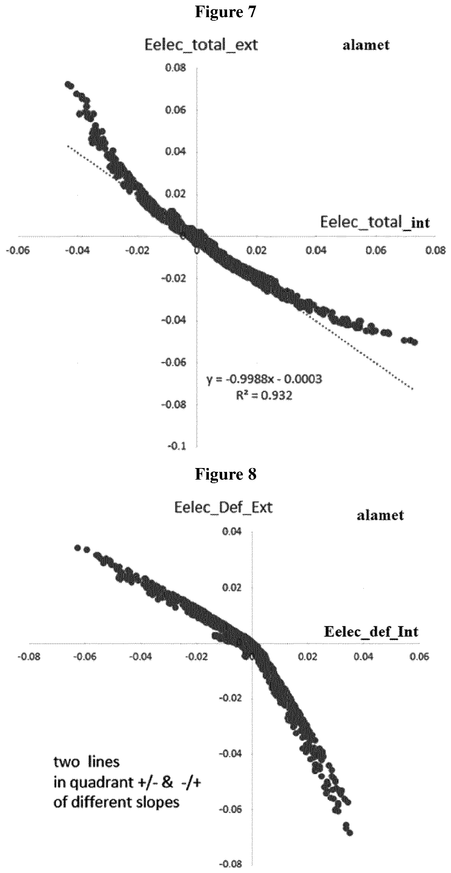

The deformation electrostatic potential (Pot_Def) has generally stronger negative values in magnitude than positive values. This is in particular due to the fact that areas of negative deformation electron density areas (electron accumulation) are spatially more concentrated than positive areas (electron depletion). As a result, the (Eelec_int, Eelec_ext) graphs may show different slopes in the +/- and -/+ quadrants (figure 8).

Due to this possible break in slope, the calculation of a correlation coefficient on the data (Eelec_int, Eelec_ext) may not properly account for the complementarity of the electrostatics of the interacting molecular entities.

By rectifying the data, a similar average slope can be obtained in the +/- and -/+ quadrants. In the case of E_elec_def, the transformation can be linear:

$$E\_elec\_int\_modified = K * E\_elec\_int \text{ if } Eelec\_int > 0; \text{ and } E\_elec\_ext\_modified = K * E\_elec\_ext$$
$$\text{if } Eelec\_ext > 0.$$

The coefficient K can be obtained in order to have the best anti-correlation and visually identical slopes. The same correction can be applied to the potential graph (pot_def_int, pot_def_ext).

The scatterplot (Eelec_tot_int, Eelec_tot_ext) of the density of total electrostatic potential (ESP) can yield, for complexes of good stability, a cloud of points with a crescent shape, as for example shown in Figure 7. This is in particular due to strong interactions at short distance (hydrogen bonds) where the electrostatic potential and energy takes large positive values on the hydrogen bond donor side while on the electronegative acceptor side the potential is only weakly negative or even slightly positive, because the Hirshfeld surface points are close to the acceptor nucleus, where the potential is very high and positive.

Such scatterplots can be fitted using a polynomial P function. The quality of the electrostatic complementarity can be measured by the normalized deviation D from the polynomial fit.

As mentioned above, said deviation D can be computed as $D_1$ or $D_2$, as follows:

$$D_2 = [ \Sigma i [ (Ei - Ei,fit)^2 / \Sigma i \ Ei^2 \ ]^{\frac{1}{2}} \quad or \quad D_1 = \Sigma i \ abs(Ei - Ei,fit) / \Sigma i \ abs(Ei)$$

To avoid that the ESP on the Hirshfeld surface near strong short contacts (hydrogen bonds) on the electronegative atom side is only weakly negative or even positive, the total ESP can be replaced by the deformation ESP, where the nucleus and core electron density are not contributing. The corresponding E_elec_def is obtained by the product of the (total, spherical or neutral-spherical) electron density multiplied by the deformation ESP. In the scatterplots of E_elec_def, the non-linear effects arising for short contacts disappear. For attractive complexes, the scatterplot takes a shape close to straight segment y = -x but may look more closely to the reunion of two half segments of negative slope, as for example shown in Figure 8, the slope being more negative in the (+,-) quadrant than in the (-,+) quadrant. As a result, the E_elec_def negative values are globally larger in magnitude than the positive values. The deformation potential has also been used in the case of a tetrachloroquinone crystal as published by Molcanov, K. et al., Crystal Growth & Design 2018, 19(1), 391-402, and gave excellent anticorrelation close to -1.

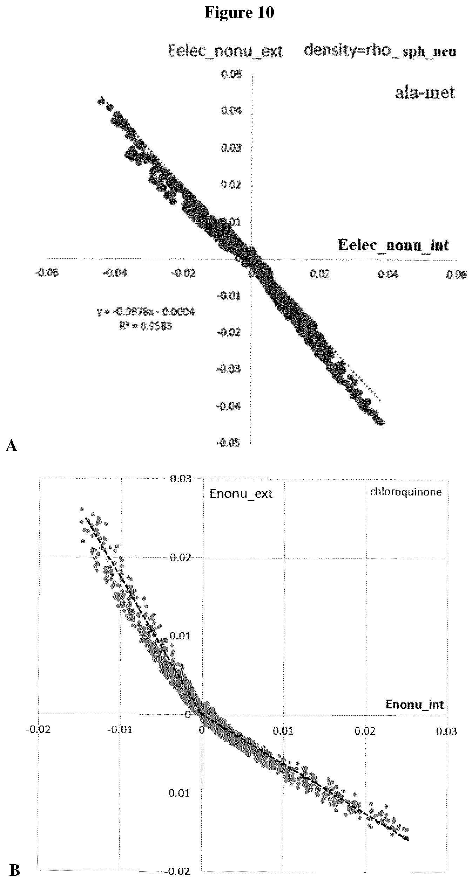

Another way to avoid, if needed, that the ESP and EED on the Hirshfeld surface near electronegative atoms is only weakly negative or even positive, is for example to replace the total ESP by the no-nucleus ESP, where the nucleus point charge is removed and replaced by a positive spherical density proportional to the core electron density of atoms. The corresponding E_elec_nonuc is obtained by the product of the (total, spherical or spherical-neutral) electron density multiplied by the nonuc ESP. In the scatterplots of E_elec_nonuc, the non-linear effects arising for short contacts are strongly attenuated. For attractive complexes with good electrostatic complementarity, the scatterplot can take a shape close to straight segment y = -x such as in in Figure 10A or look more generally closely to the reunion of two half lines of negative slope, as for example shown in Figure 10B, the slope being more negative in the (-,+) quadrant than in the (+,-) quadrant. As a result, the E_elec_nonuc positive values are larger in magnitude than the negative values, but to a much lesser extent than the E_elec_tot values.

The E_elec scatterplot of an attractive complex with good electrostatic complementarity can be rendered close to a straight segment y=-x by using a linear combination of E_elec_tot and E_elec_def or of E_elec_nonuc and E_elec_def (see for instance example 1). Such linear combination of E_elec_tot and E_elec_def or of E_elec_nonuc and E_elec_def are hereafter called "linearized ESP" and "linearized EED" respectively. The linear fit and the correlation coefficient R performed on the linearized EED scatterplot may then become a tool to measure in one number the quality of a complex electrostatic complementarity.

For example, a linear combination of 12% of E_elec_tot and of 88% of E_elec_def may be used for the int and ext values, in particular for the analysis of a streptavidin / biotin complex (example 2). In particular, linear combinations of E_elec_nonuc and E_elec_def can also be used. In particular, a linear combination of 20% of E_elec_nonuc and of 80% of E_elec_def has been used for the int and ext values, or a linear combination of 15% of E_elec_nonuc and of 85% of E_elec_def, as for example in the case of the analysis of a DL-histidine crystal (Edington, P. et al., Acta Crystallographica Section B 1974, 30(1), 204-206).

Linear combinations of E_elec_nonuc and E_elec_def have also been used in the case of a serine crystal packing (as described by Dittrich, B. et al., Acta Crystallographica Section A: Foundations of Crystallography 2005, 61(3), 314-320). They all rendered the scatterplot even more linear, maximizing the $R^2$ coefficient, for example with 75% of E_elec_nonuc and of 25% of E_elec_def.

These combinations may have, if needed, less non linear effects on short contacts on the Hirshfeld surface.

Other linear combinations can be performed, such as linear combinations regarding the total and deformation potential, for example:

$$\text{Pot\_int} = 0.63\ \text{Pot\_Def\_int} + 0.37\ \text{Pot\_Tot\_int};$$

$$\text{Pot\_ext} = 0.39\ \text{Pot\_Def\_ext} + 0.61\ \text{Pot\_Tot\_ext}$$

in particular for the analysis of an alanine-methionine molecule crystal structure (example 4).

In a particular embodiment, the describing of step (iv) is performed by computing the linear correlation coefficient R of said "linearized EEP" plurality of points, or of part of said plurality of points.

R can be obtained by summation over the plurality of points i, or over part of said plurality of points :

$$R = \Sigma_i\, [\ \text{E\_elec\_int}(i) * \text{E\_elec\_ext}(i)\ ]\, /\, [\ [\Sigma_i\, \text{E\_elec\_int}(i)^2\,] * [\Sigma_i\, \text{E\_elec\_ext}(i)]\ ]^{\frac{1}{2}}$$

In a particular embodiment, the describing of step (iv) is performed by (a) fitting the simple linear regression line of said "linearized EEP" plurality of points, or of part of said plurality of points, and (b) displaying a representation of said surface indicating for the plurality of points, or part of it, the deviation from the regression line.

The electrostatic complementarity can be assessed more generally by other descriptors than the EES. For example the descriptor F=ESP*(electron density)$^p$, with p between 0 and 1, products of the electrostatic potential and of power *p* of the electron density can be used to assess the quality of electrostatic complementarity.

All the subject-matter and embodiments wherein the EED descriptor is replaced by the F descriptor are thus also part of the present specification.

For example, the descriptor $F_{\frac{1}{2}}$ can, if needed, gives more weight to the weak contacts (regions of lower electron density) than the standard descriptor $F_1$ = EED. This has for instance be obtained in the case of a (+)-epi-biotin / streptavidin complex and of dl-His crystal (Figure 18)

When the electrostatic match is assessed between two entities which are not electrically neutral, the scatterplot (EES_int EES_ext) will not be balanced in the number of EES values in the negative and positive regions while the magnitudes of the minimum and maximum will also be dissimilar.

This can, if necessary, be compensated by different methods:

- The inner and/or exterior ESP values may be shifted by addition of a constant value in order to have a mean or a median value equal to zero;
- The inner and/or exterior ESP values may be shifted by addition of a constant value in order to have a maximum and minimum values of same magnitude, in absolute value;
- The global charge of the molecule and/or of the environment may be changed and set for example electrically neutral;
- The Hirshfeld surface defined by equation H = ½ , may be shifted towards the exterior (H=a < ½). This will shift the inner and exterior EED to smaller and larger values, respectively;
- The Hirshfeld surface defined by equation H = ½, may be shifted towards the interior (H=a > ½). This will shift the inner and exterior EED to larger and smaller values, respectively;
- In case the environment is charged, a selection of atoms may be retained only in order to obtain an entity which is close to electroneutrality. For example, in the case of a protein environment, a subset of atoms in the binding site located up to a certain distance to the inner molecule may be selected.

The use of a Hirshfeld surface derived from an electron density using spherical neutral atoms instead of multipolar charged atoms (total electron density) can reduce non-linear effects in the total_EED scatterplots occurring for polar hydrogen atoms (bound to oxygen or nitrogen) involved in strong hydrogen bonds.

In an analysis of a complex, more information than just the interior and exterior EED values on the Hirshfeld surface can be retrieved, in particular the inner and outer chemical species which are closest to the surface point or which are contributing most to the electron density. The atoms of the molecule and of the environment making a contact can also be retrieved.

In the case of systems where the environment is a biopolymer such as a protein, when the solvent surrounding the ligand molecule has been totally modelled, including hydrogen atoms, the Hirshfeld surface may be computed between the ligand molecule and the environment constituted by the protein and the solvent.

In the case of systems where the environment is a biopolymer such as a protein, the Hirshfeld surface may be computed between the ligand molecule and the environment constituted by the protein, the regions interacting with the solvent can be omitted by using cutoffs. For example, the electron density on the Hirshfeld surface should be larger than a threshold (typically RHO > 0.0013 e/Å$^3$). Alternatively, surface points which are at a distance larger the van der Waals radius of the protein atoms plus typically 1 angstrom may be omitted.

In the case of systems where the environment is a biopolymer such as a protein or nucleic acid, the Hirshfeld surface may be computed between the ligand molecule and the environment constituted by the biopolymer. The surface regions interacting with the solvent may be omitted by imposing that the exterior electron density takes always a value larger than a threshold (typically RHO > RHOlim = 0.0013 e/Å$^3$).

The scatterplot of linearized EED of a complex displays typically points which are located on several half straight segments starting from the origin (0,0). Each half line of points represents an intermolecular contact. The points with largest EED values in magnitude are located in the central area of the contact (around the interatomic straight line). The points with lower EED values, i.e. closer to the origin are located on the periphery of the interatomic contact.

The half straight segments in the linearized EED scatterplot can be directly related to the intermolecular contacts present in the complex as the points can be identified according to the interior and exterior chemical species or according to the concerned atoms in the molecule and its environment.

The half straight segments in the linearized EED scatterplot which are long correspond to strong electrostatic interactions while the shorter fragments correspond to weaker electrostatic interactions.

The half straight segments in the (+,-) quadrant of the scatterplot of linearized (EED_int,EED,ext) which have a negative slope significantly smaller than one in magnitude, typically between -0.75 and -0.3, correspond to interatomic contacts between a highly electropositive atom or group and a weakly electronegative atom or group.

The half straight segments in the (+,-) quadrant of the scatterplot of linearized (EED_int,EED,ext) which have a negative slope significantly larger than one in magnitude, typically between -1.3 and -3. correspond to interatomic contacts between a weakly electropositive atom or group and a highly electronegative atom or group.

The half straight segments in the (-,+) quadrant of the scatterplot of linearized (EED_int,EED,ext) which have a negative slope significantly smaller than one in magnitude, typically between -0.75 and -0.3, correspond to interatomic contacts between a strongly electronegative atom or group and a weakly electropositive atom or group.

The half straight segments in the (-,+) quadrant of the scatterplot of linearized (EED_int,EED,ext) which have a negative slope significantly larger than one in magnitude, typically between -1.3 and -3. correspond to interatomic contacts between a weakly electronegative atom or group and a strongly electropositive atom or group.

The scatterplot of linearized (EED_int,EED,ext) can be modelled as a superposition of half straight segments, for which parameters are the slope and the EED_int extremum. This modelling could be used in further data processing

The EED interior and exterior can be used to color the Hirshfeld surface around the said molecule and highlight the electropositive and electronegative areas of the molecule and its environment.

The linearized (EED_int,EED,ext) set of points can be used to compute a discrepancy function Delta = EEDint + EED_ext. The Hirshfeld surface colored according to the discrepancy function will highlight the intermolecular contacts which are not favorable or not optimal. Regions with large positive Delta values correspond to interacting atoms or chemical groups which are both electropositive, or alternatively, one strongly electropositive and the other only weakly electronegative. Regions with large negative Delta values correspond to interacting atoms or chemical groups which are both electronegative, or alternatively one strongly electronegative and the other only weakly electropositive.

In the case of a crystal structure prediction of a small molecule, the scatterplot and correlation coefficient of linearized (EED_int,EED,ext) graph computed on the molecule and its close crystal environment can be used to filter out non-optimal crystal packings. Correlation coefficient far from -1 and half straight lines in the quadrants (+,+) and (-,-) denote the presence of contacts which are unfavorable from an electrostatic point of view.

In the case of a drug design project, to improve the affinity of a ligand molecule to a protein binding pocket, the scatterplot and correlation coefficient of linearized (EED_int,EED,ext) graph computed on the molecule and its protein environment can be used to identify favorable and unfavorable contacts around the ligand and modify the ligand accordingly. Correlation coefficient far from -1 and half straight lines in the quadrants (+,+) and (-,-) denote the presence of contacts which are unfavorable from an electrostatic point of view.

In the case of a structure determination of a protein/ligand complex by cryo electron diffraction, the scatterplot and correlation coefficient of linearized (EED_int,EED,ext) graph computed on the ligand and its protein environment can be used to analyze the favorable and unfavorable contacts around the ligand and help model the correct ligand positioning. Correlation coefficient far from -1 and half straight lines in the quadrants (+,+) and (-,-) denote the presence of contacts which are unfavorable from an electrostatic point of view.

In the case of protein engineering in order to improve the ligand affinity to a protein binding pocket, the scatterplot and correlation coefficient of linearized (EED_int,EED,ext) graph computed on the bound molecule and its close macromolecular environment can be used to identify favorable and unfavorable contacts around the ligand and to design mutations on the protein. Correlation coefficients far from -1 and half straight lines in the quadrants (+,+) and (-,-) denote the presence of contacts which are unfavorable from an electrostatic point of view.

## FIGURES

**Figure 1** presents a (E_elec_int, E_elec_ext) graph representing the electrostatic energy density on the molecular surface corresponding to the real aminosalicylic acid (AMSA) crystal structure according to example 1. Unit: $(e/Å)*(e/Å^3) = e^2/Å^4$. The atom coordinates are described in the following reference: Meenashi, R., Selvaraju, K., Stephen, A. D., & Jelsch, C. (2020). Journal of Molecular Structure, 1213, 128139.

**Figure 2** presents a (Vint,Vext) graph (being not part of the invention), wherein Vint is the electrostatic potential generated by the AMSA molecule, and Vext is the electrostatic potential generated by the neighboring molecules, according to example 1. Unit: e/Å.

**Figure 3** presents a (E_elec_int, E_elec_ext) graph of a close to real predicted crystal of AMSA molecule crystal according to example 3.

**Figure 4** presents a (E_elec_int, E_elec_ext) graph of a true negative predicted structure of AMSA molecule as mentioned in example 3.

**Figure 5** corresponds to a (E_elec_def _int , E_elec_def_ext) scatter plot that has been obtained for the crystal structure of guanine according to example 3.

**Figure 6** corresponds to a (E_elec_def_int , E_elec_def_ext) scatter plot corrected by a multiplication coefficient of 1.3 for positive values of E_elec_def _int and E_elec_def_ext, according to example 3.

**Figure 7** presents a (E_elec_Total_int, E_elec_Total_ext) scatter plot obtained for the dl-alanine-methionine crystal structure according to example 4 using the total potential.

**Figure 8** presents a (E_elec_Def_int, E_elec_Def_ext) scatter plot obtained for an alanine-methionine molecule crystal structure according to example 4 using the deformation potential.

**Figure 9** presents a linearization of a (E_elec_Def_int, E_elec_Def_ext) graph by modification of high positive values according to AlaMet crystal, example 4.

**Figure 10A** presents a (E_elec_nonuc_int, E_elec_nonuc_ext) scatter plot obtained for an alanine-methionine molecule crystal structure according to example 4 using the nonuc potential.

**Figure 10B** presents a (E_elec_nonuc_int, E_elec_nonuc_ext) scatterplot obtained for tetrachloroquinone crystal structure using the nonuc potential.

**Figure 11** presents the (E_elec_int, E_elec_ext) scatter plot obtained for AMSA molecule experimental crystal structure as for instance mentioned in example 1. The longest straight segment in the (+,-) quadrant corresponds to the O-H...O strong hydrogen bond between two neighboring carboxylic acids. The points associated to this interaction are highlighted in black.

**Figure 12** presents a (Vint,Vext) graph (being not part of the invention), wherein Vint is the electrostatic potential generated by an aceclofenac molecule, and Vext is the electrostatic potential generated by the neighboring molecules of the crystal, according to example 5. Unit: e/Å.

**Figure 13** presents a (E_elec_int, E_elec_ext) graph for aceclofenac crystal according to the invention, as described in example 5.

**Figure 14** illustrates a linear combination regarding the total and deformation potential for the analysis of an alanine-methionine molecule crystal structure as described in example 4.

**Figure 15** concerns the Pot_def scatterplot (not part of the invention) of a metformin chloride salt according to claim 6.

**Figure 16** deals with the EED_def scatterplot of the invention of a metformin chloride salt according to claim 6.

**Figure 17** presents the study of 4 poses of a Human Cytochrome P450 (CYP) 2D6 - Prinomastat complex according to example 7. The experimental pose is that noted docking #5.

**Figure 18** presents the (F_int,F_ext) scatterplot for DL-Histidine crystal. F is the square root of the electron density multiplied by the electrostatic potential. The C...C contacts are highlighted in black.

## EXAMPLES

### Example 1: Study of aminosalicylic acid (AMSA) crystalline structures

The reference structure was described by Meenashi, R. et al. Journal of Molecular Structure 2020, 1213, 128139.

The modelling of electron density and multipolar electrostatic potential is described in the article. After database transfer, the molecule was set electrically neutral.

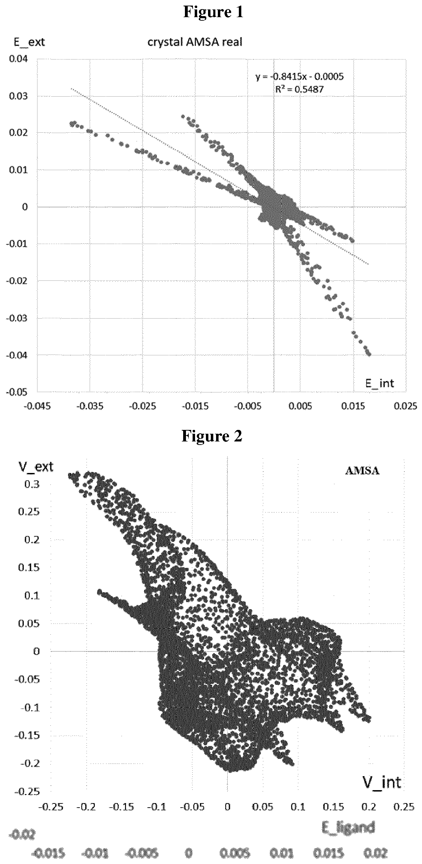

(E_elec_int, E_elec_ext) graphs representing the electrostatic energy density on the molecular surface corresponding to the real AMSA crystal structure have been produced according to the invention (**figure 1**).

(Vint,Vext) graphs (being not part of the invention), wherein Vint is the electrostatic potential generated by the molecule, and Vext is the electrostatic potential generated by the neighboring molecules, have also been produced for the same real crystal (**figure 2**).

It is clear from these figures that the (Vint,Vext) graphs do not give a clear mapping of the crystal electrostatic interactions. This unclear mapping of the crystal contacts does not clearly identify contacts that are favourable and those that are unfavourable.

In sharp contrast, the (E_elec_int, E_elec_ext) graphs according to the invention displays a scatter plot that is composed of four straight segments which are not far from the diagonal y=-x. The points are essentially in the (+,-) and (-,+) quadrants, showing a good EED anti-correlation for the real crystal.

Two strong electrostatic interactions: Hydrogen bonds O-H...O(carboxylic_acid) and N-H...O(phenol), have been identified. The O-H...O interaction has a longer corresponding segment as it is stronger than the N-H...O hydrogen bond.

In addition, AMSA crystal structures which have been predicted by the MOLPAK software (*ibid*.) have been studied.

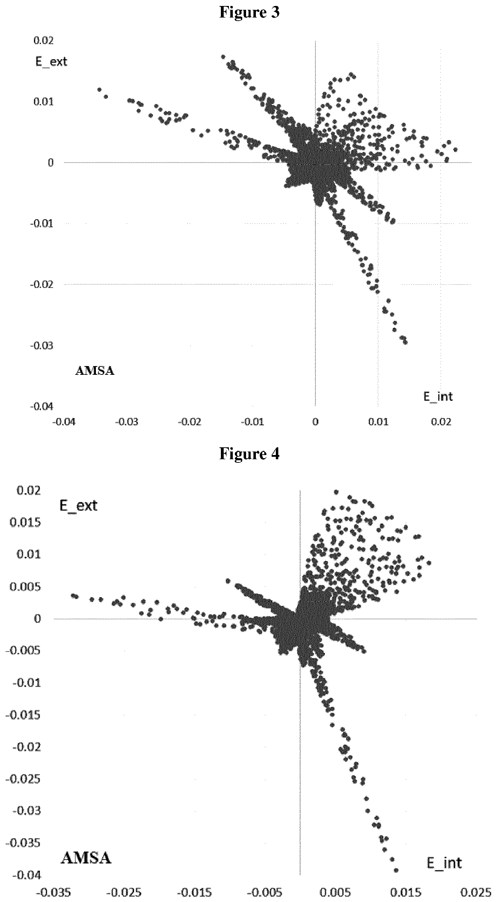

For example, the (E_elec_int, E_elec_ext) graphs of a close to real structure is shown in **figure 3**. This diagram shows the same four segments in the (+,-) and (-,+) quadrants corresponding to favorable electrostatic contacts. The diagram displays also some unfavorable interactions in the (+,+) quadrant, which correspond to the fact that the studied structure is not the real structure but a close to real structure.

The (E_elec_int, E_elec_ext) graph of a true wrong predicted crystal structure (**figure 4**) displays such two straight lines in the (+,-) and (-,+) quadrants but they are very far from the diagonal y=-x. Morevover the diagram shows a large number of points with large EED values in the (+,+) quadrant corresponding to repulsive electrostatic interactions.

Considering a crystal packing having favorable electrostatic contacts, it is noted that a linearized scatter plot could be obtained by considering the deformation and "nonuc" potentials.

A linear combination of 30% of E_elec_nonuc and of 70% of E_elec_def was used in the case of AMSA molecule to linearize the scatter plot of crystal packings having favorable electrostatic contacts (figures 1, 2, 3 , 4), as follows:

$$Pot\_comb = 0.7\ Pot\_Def\ +\ 0.3 * Pot\_nonuc.$$

$$Ecomb = \ total\_spherical\_neutral\_density * Pot\_comb.$$

### Example 2: Study of streptavidin /biotin docked poses

The E_elec scatterplot of an attractive complex with good electrostatic complementarity can be rendered close to a straight segment y=-x by using a linear combination of E_elec_tot and E_elec_def or of E_elec_nonuc and E_elec_def.

In this example, a linear combination of 12% of E_elec_tot and of 88% of E_elec_def has been used for the analysis of streptavidin /biotin docked poses obtained with the program GOLD.

The linear fit and the correlation coefficient R has been performed on the linearized EED scatterplot obtained as described above.

A coefficient R of -1 would correspond to an ideal score. This correlation coefficient R enabled to measure the quality of a complex electrostatic complementarity.

Indeed, a coefficient R such as:

- $R^2$ = 0,85 (corresponding to $R$ = -0,92) has been obtained for the real structure;

- $R^2 = 0,77$ (corresponding to $R = -0,88$) has been obtained for a docking pose close to the real structure;
- $R^2 = 0,05$ to $0,59$ (corresponding to $R = -0,22$ to $-0,77$) has been obtained for docking poses that are far to the real structure.

**Example 3: Study of the guanine crystalline structure**

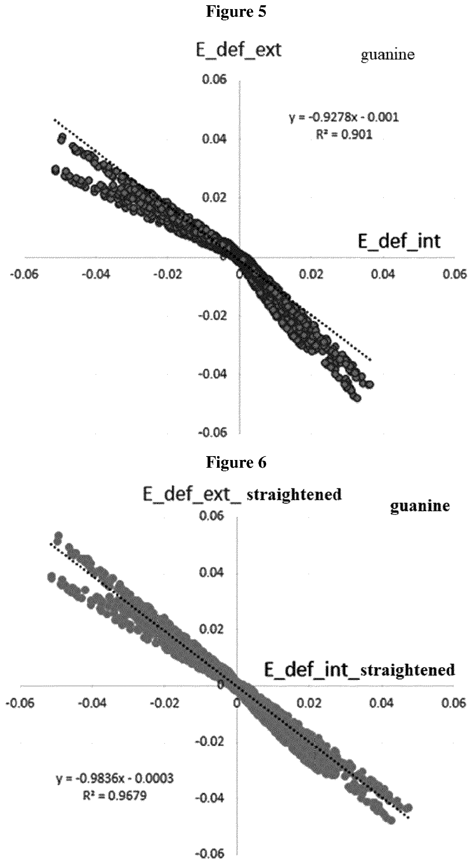

A (E_elec_def _int , E_elec_def_ext) scatter plot has been obtained for a guanine crystal structure (**figure 5**). A slope break at the origin can be observed.

E_elec_def was then corrected by a multiplication coefficient of 1.3 for positive values of E_elec_def _int and E_def_ext.

This makes it possible to obtain a scatter plot, whose slopes are similar in the +/- and -/+ quadrants (**figure 6**).

A correlation coefficient could then be calculated directly on the rectified data, which is a direct measurement of the complementarity and which reaches -0.984 in the case of the guanine crystal.

(Electron density model transferred from the ELMAM2 database, electrostatic potential and electron density values projected onto the Hirshfeld (electrostatic potential and electron density values projected on the Hirshfeld surface from spherical and neutral atoms)).

**Example 4: Study of an alanine-methionine crystalline structure**

An alanine-methionine molecule crystal structure has been studied by methods of the invention.

A (E_elec_total_int , E_elec_total_ext) scatter plot has been obtained according to the invention (**figure 7**). The scheme displays a very good electrostatic complementarity. But this electrostatic complementarity could not be optimally measured by a correlation coefficient due to the crescent shape.

A graph with two half straight lines could be obtained using the deformation potential (**figure 8**).

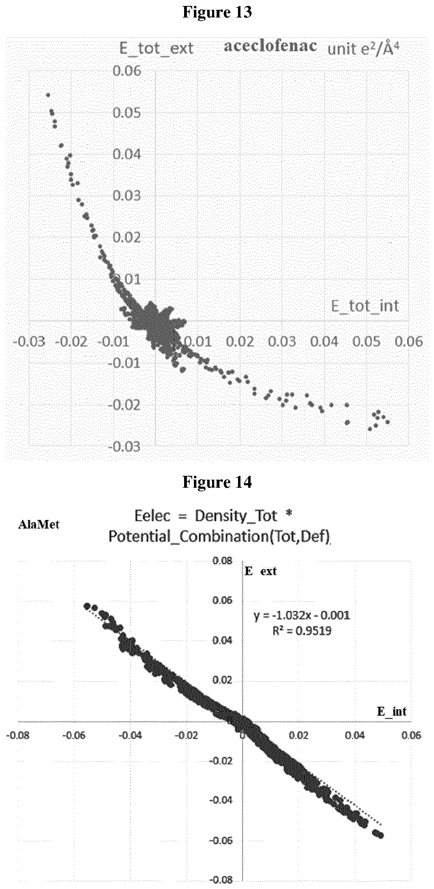

A linear combination of total potential and deformation potential has been considered to obtain Pot_int_average $= 0$ and Pot_ext_average $= 0$ (on Hirshfed surface), and similar slope in the (+,-) and (-,+) quadrants (**figure 14**), as follows:

$$\text{Pot\_int\_comb} = 0.63 \, \text{Pot\_Def\_int} + 0.37 \, \text{Pot\_Tot\_int}$$

$$\text{Pot\_Ext\_comb} = 0.39 \, \text{Pot\_Def\_ext} + 0.61 \, \text{Pot\_Tot\_ext}$$

The combination of EEDs yields graphs close to linearity (slope +/- = slope -/+ ). A measure of electrostatic complementarity can then be the correlation coefficient (E_elec_comb_int,E_elec_comb_ext). For a given molecule, the combination found can be used for evaluation of predicted crystal structures.

A linearization could be obtained by this method when considering other molecules, for instance the guanine molecule of example 3.

A linearization of Eelec_int/ext graph of AlaMet crystal has been obtained by modification of high positive values as follows:

$$\text{E\_modif} = \text{E\_elec} \qquad \text{if} \quad \text{Elec} < 0.02 \ e^2/\mathring{A}^3$$

$$\text{E\_modif} = 0.02 * (\text{E\_elec}/0.02)^{0.75} \quad \text{if} \quad \text{Elec} > 0.02 \ e^2/\mathring{A}^3$$

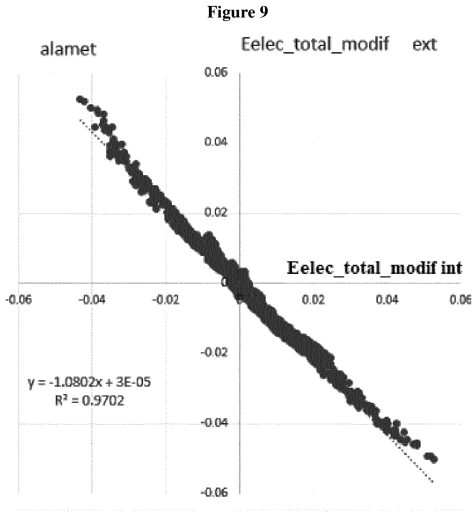

Results are presented in **figure 9**.

It is noted that a linear scatter plot can also be obtained for AlaMet crystal by considering the nonuc potential (**figure 10A**), such as:

$$\text{Charge\_density} = \text{Nucleus} + \text{Core} + \text{Valence} = Z.\delta_{nuc} + Nc \, \rho_{core} + Nv \, \rho_{valence}$$

Replaced by: $\rho_{nonuc} = (Nc\text{-}Z) \, \rho_{core} + Nv \, \rho_{valence}$ where p is the atomic electron density.

The derived potential_ nonuc and corresponding E_elec_nonuc have been computed accordingly.

The no nucleus (nonuc) is thus the replacement of nucleus by core Electron Density and is suitable to attenuate the high positive potential near the nucleus and the non-linear effects observed in EED graphs for close contacts.

Good Electrostatic complementarity is observed by a cloud close to the line $E_{ext} = - E_{int}$ In the present case of a AlaMet crystal, $|minimum| \cong$ maximum Potential or Eelec and a slope close to -1 in both quadrants (+,-) and (-,+) have been observed.

**Example 5: Study of the aceclofenac molecule crystal structure**

The crystal structure of the aceclofenac molecule was described by Jelsch, C. et al., Journal of Molecular Structure 2020, 1205, 127600.
The electrostatic complementarity represented by the inner and outer ESP on the Hirshfeld surface (not part of the invention) is presented in **figure 12**.
The EED according to the invention was obtained as follows:

$$EED\_tot\_int = density\_int * Pot\_tot\_ext;$$

and

$$EED\_tot\_ext = density\_ext * Pot\_tot\_int.$$

It is underlined that the interior and exterior density is the same on the Hirshfeld surface. Therefore it can be written:

$$EED\_tot\_int = density * Pot\_tot\_int;$$

and

$$EED\_tot\_ext = density * Pot\_tot\_ext.$$

The complementarity does not appear clearly in the (Pot_Int,Pot_Ext) graph, with a large cloud of points around the origin and two groups of points with large positive values, representing two reciprocal O-H...O strong hydrogen bonds.
On the contrary, the scatterplot of EED of the invention (**figure 13**) permits to better identify the strong and weaks contacts of aceclofac in the crystal packing. A group of points going outwards represents each a specific contacts.

**Example 6: Study of crystals of salts**

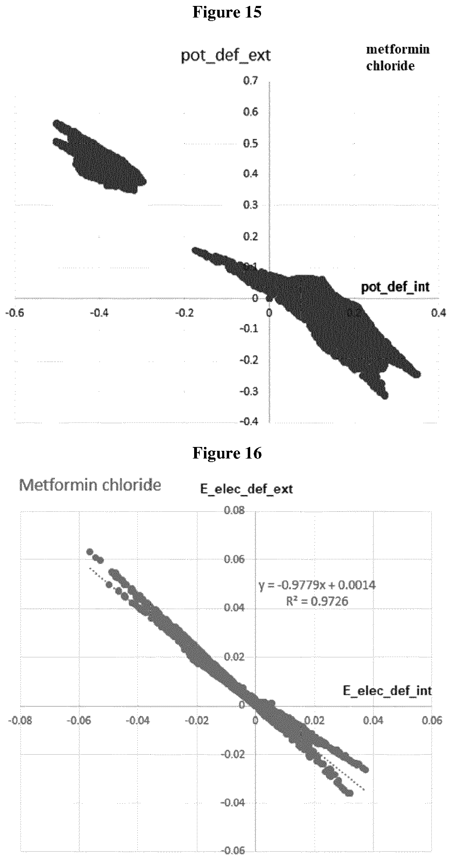

Crystal of metformin chloride salt (an example of charged systems) has been studied.
The Hirshfeld surface was first generated around a Cl⁻ anion and a metformin cation not in contact in the crystal.
The Pot_nonuc scatterplot (not part of the invention) shows the surfaces around Cl⁻ and met⁺ as two separate groups groups (**figure 15**).
The EED_def scatterplot of the invention shows in contrast that the two clusters are now joined by the regions around the origin with weaker EED (**figure 16**). Here the EED_def descriptor yields a nearly linear graph and the anticorrelation of EED_def is very good. The chloride anion forms 4 strong Cl⁻... H-N strong hydrogen bonds which correspond to the regions of the EED graph with largest magnitudes.
The Hirshfeld surface was also computed around only the metformin cation, which is positively charged. The computation of the ESP and of EED around a charged moiety resulted in an unbalanced property whose average value on the Hirshfeld surface is here positive. To have a balanced ESP, it can be subtracted from the potential its average value on the Hirshfeld molecular surface. This can be done on the interior and exterior ESP. The resulting modified EED highlights then the electrostatic complementarity.
A balanced ESP could also be obtained by neutralizing the metformin molecule and neutralizing the surrounding entity. The resulting modified interior ESP is balanced. The resulting ESP_ext is still negative on average because there are several chloride anions in the immediate environment of the metformin cation. Its average value needs still to be subtracted in order to have a balanced ESP_ext. The resulting EEP diagram is centered around the origin and shows balanced electrostatic complementarity around the charge metformin molecule. The non-balanced EEP diagram is similar with a good correlation but is mostly located in the (+,-) quadrant.

**Example 7: Ligand-Receptor complex poses study**

10 ligand poses of the Human Cytochrome P450 (CYP) 2D6 - Prinomastat complex were generated and mixed with the true structure, for a blind test.

The ESP_nonuc and EED_nonuc were computed for the protein/ligand Hirshfeld interface. Protein structure was retrieved from the Protein Databank, code 3qm4. Water molecules were removed and the hydrogen atoms were added to the structure. The charge density of the ligand and of the porphyrin was multipolar, transfered from the ELMAM2 database (Domagala et al., 2012). The charges of the protein atoms were transfered from the AMBER all_amino03 dictionary of point charges. The derived nonuc ESP was computed for both ligand and protein.

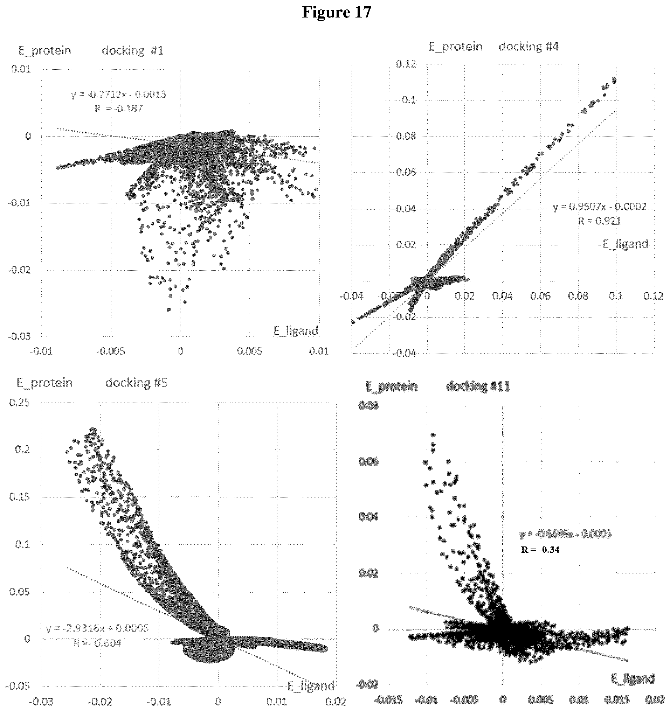

Four of the poses are illustrated in figure 17.

The (EED_ligand,EED_protein) scatterplots reveals only two favorables poses among the eleven complexes: Poses 5 and 11.

The pose #5 has the strongest negative correlation coefficient r = -0.60 and shows therefore the best electrostatic complementarity and it is indeed the experimental structure. The strong contact in the (-,+) quadrant corresponds to the coordination of the electronegative nitrogen atom on the ligand pyridine ring with the positively charged iron atom on the porphyrin moiety of the protein. Pose #4 is dominated by an unfavorable contact between Fe atom of the porphyrin group of the protein and a H-C hydrogen atom, both are electropositive and distance is short d=1.95 Å.

Pose #1 is also unfavorable.

**Claims**

1. A computer based method for evaluating the stability of at least one structure of at least one complex constituted of a molecule and its environment, comprising the following steps of:

   (i) Receiving at least one structure of at least one complex constituted of a molecule and its environment;
   (ii) Defining from the structure received in step (i) a surface delimiting said molecule from said environment;
   (iii) Determining for each point of a plurality of points of the surface defined in step (ii) the electrostatic energy density E_elec_int computed as the product of the electrostatic potential (ESP) generated by said molecule and the electron density generated by said environment, and the electrostatic energy density E_elec_ext computed as the product of the electrostatic potential generated by said environment and the electron density generated by said molecule, or the descriptor F_int computed as the product of the electrostatic potential (ESP) generated by said molecule and (electron density)$^p$, with p between 0 and 1, generated by said environment, and the descriptor F_ext computed as the product of the electrostatic potential generated by said environment and (electron density)$^p$, with p between 0 and 1, generated by said molecule.

2. The computer based method according to claim 1, comprising the following steps of:

   (i) Receiving at least one structure of at least one complex constituted of a molecule and its environment;
   (ii) Defining from the structure received in step (i) a surface delimiting said molecule from said environment;
   (iii) Determining for each point of a plurality of points of the surface defined in step (ii) the electrostatic energy density E_elec_int computed as the product of the electrostatic potential (ESP) generated by said molecule and the electron density generated by said environment, and the electrostatic energy density E_elec_ext computed as the product of the electrostatic potential generated by said environment and the electron density generated by said molecule, or the descriptor F_int computed as the product of the electrostatic potential (ESP) generated by said molecule and (electron density)$^p$, with p between 0 and 1, generated by said environment, and the descriptor F_ext computed as the product of the electrostatic potential generated by said environment and (electron density)$^p$, with p between 0 and 1, generated by said molecule;
   (iv) Estimating for the at least one structure of the at least one complex the complementarity of said molecule with said environment by describing the plurality of points according to their (E_elec_int, E_elec_ext) or (F_int, F_ext) couple of values as determined in step (iii).

3. The computer based method according to claim 1 or 2, wherein the molecule is an organic compound, an organometallic compound, or an inorganic compound, having a molecular weight of less than 5000 or 10000 Da, in particular:

   - an organic compound, an organometallic compound, or an inorganic compound, having a molecular weight of less than 900 Da, or
   - a peptide or a foldamer, in particular having a molecular weight between 900 and 5000 or 10000 Da;

or a biopolymer, in particular a protein or a nucleic acid.

4.  The computer based method according to anyone of claim 1 to 3, wherein the environment is:

    - constituted of or comprises a biopolymer, in particular a protein or a nucleic acid;
    - constituted of or comprises a plurality of said molecule, said molecule being in particular an organic compound, an organometallic compound, or an inorganic compound, having a molecular weight of less than 5000 or 10000 Da; or
    - constituted of said molecule, said molecule being in particular an organic compound, an organometallic compound, or an inorganic compound, having a molecular weight of less than 5000 or 10000 Da.

5.  The computer based method according to claim 1 or 2, wherein the complex is:

    - a ligand-biopolymer complex, in particular a ligand-protein complex, wherein the ligand is more particularly an organic compound having a molecular weight lower than 5000 or 10000 Da, even more particularly an organic compound having a molecular weight lower than 900 Da or an organic compound having a molecular weight between 900 and 5000 or 10000 Da;
    - a crystalline structure, in particular a crystal packing, in particular constituted of a molecule and of its environment of molecules of the same nature, said molecule being more particularly an organic compound, an organometallic compound, or an inorganic compound, having a molecular weight of less than 5000 or 10000 Da;
    - a metal organic framework (MOF), in particular constituted of a small molecule and of its MOF environment, said molecule being more particularly an organic compound, an organometallic compound, or an inorganic compound, having a molecular weight of less than 5000 or 10000 Da;
    - made of an inorganic porous matrix and a small molecule, the inorganic porous matrix being in particular a zeolite and said small molecule being more particularly an organic compound, an organometallic compound, or an inorganic compound, having a molecular weight of less than 10000 Da; or
    - an assembly of two biopolymers, said biopolymers being in particular proteins or a nucleic acids.

6.  The computer based method according to anyone of preceding claims, wherein the surface defined in step (ii) is:

    - a Hirshfeld surface between the molecule and its environment, a shell of space around said Hirshfeld surface, a Hirshfeld surface shifted toward the interior, or a Hirshfeld surface shifted towards the environment;
    - an equidistance surface between the molecule and the environment, a shell of space around said equidistance surface, an equidistance surface shifted toward the interior, or an equidistance surface shifted towards the environment; or
    - an equidistance-over-van-der-Waals-radius surface between the molecule and the environment, a shell of space around said surface, a surface shifted toward the interior, or a surface shifted towards the environment.

7.  The computer based method according to anyone of preceding claims, wherein the electrostatic potential is:

    - The total electrostatic potential generated by the molecule;
    - The deformation electrostatic potential, corresponding to the difference between the total electrostatic potential and the theoretical electrostatic potential generated by atoms all with zero charge and spherical electron density;
    - The spherical electrostatic potential, corresponding to the total electrostatic potential without any multipolar component;
    - The spherical deformation electrostatic potential, corresponding to the difference between the spherical electrostatic potential and the theoretical electrostatic potential generated by atoms all with zero charge and spherical electron density;
    - The spherical deformation electrostatic potential, corresponding to the difference between the spherical electrostatic potential and the theoretical electrostatic potential generated by atoms all with zero charge and spherical electron density;
    - The "non-nucleus" electrostatic potential, corresponding to a potential computed from a modified charge density wherein the nucleus point charge of all atoms is replaced by a positive spherical charge density $rho\_proton(r)$ centered on the nucleus which has the same distribution as a function distance r to the nucleus as $rho\_core(r)$, the atomic electron density of core electrons;
    - The "non-nucleus" spherical electrostatic potential, corresponding to a potential computed from a modified spherical charge density wherein the nucleus point charge of all atoms is replaced by a positive spherical charge density $rho\_proton(r)$ centered on the nucleus which has the same distribution as a function distance r to the nucleus as rho_core(r), the atomic electron density of core electrons; or
- The punctual electrostatic potential, corresponding to the atomic partial charges placed at the nuclei.

8. The computer based method according to anyone of preceding claims, wherein the electron density is:

- The total electron density;
- The spherical electron density, corresponding in particular to an approximation of the total electron density using a superposition of atoms with spherical electron density;
- The spherical neutral electron density, corresponding in particular to an approximation of the total electron density using a superposition of atoms with all zero charge and spherical electron density;
- The spherical electron density, corresponding in particular to an approximation of the total electron density using a superposition of atoms with spherical electron density;
- The deformation electron density, corresponding in particular to the difference between the total electron density and the theoretical electron density generated by atoms all with zero charge and spherical electron density;
- The spherical deformation electron density, corresponding in particular to the difference between the spherical electron density and the theoretical electron density generated by atoms all with zero charge and spherical electron density;
- The punctual charge density (rho_punctual), corresponding to atomic partial charges placed at the nuclei;
- The "non-nucleus" charge density corresponding to a modified charge density where the nucleus point charge of all atoms is replaced by a positive spherical charge density rho_proton(r) centered on the nucleus which has the same distribution as a function of distance r to the nucleus as rho_core(r), the atomic electron density of core electrons; or
- The "non-nucleus" spherical charge density corresponding to a modified spherical charge density where the nucleus point charge of all atoms is replaced by a positive spherical charge density rho_proton(r) centered on the nucleus which has the same distribution as a function of distance r to the nucleus as rho_core(r), the atomic electron density of core electrons.

9. The computer based method according to anyone of claims 2 to 8, wherein the plurality of points are represented in step (iv) in a 2D diagram according to:

- their (E_elec_int, E_elec_ext) or (F_int, F_ext) coordinates;
- their (E_elec_def_int, E_elec_def_ext) coordinates, wherein the electron potential is the deformation electrostatic potential;
- their (E_elec_def_sph_int, E_elec_def_sph_ext) coordinates, wherein the electron potential is the spherical deformation electrostatic potential;
- their (E_elec_nonuc_int, E_elec_nonuc_ext) coordinates, wherein the electron potential is the "non-nucleus" electrostatic potential;
- their (E_elec_nonuc_sph_int, E_elec_nonuc_sph_ext) coordinates, wherein the electron potential is the "non-nucleus" spherical electrostatic potential;
- their (E_elec_punctual_int, E_elec_punctual_ext) coordinates, wherein the electron potential is the punctual charges electrostatic potential;
- Their (E_elec_int, E_elec_ext) coordinates wherein the electrostatic energy density is a linear combination of E_elec_tot, and E_elec_def, or of E_elec_nonuc and E_elec_def.
- Their (E_elec_int, E_elec_ext) coordinates wherein the electrostatic energy density is a linear combination of E_elec_sph, and E_elec_def_sph, or of E_elec_nonuc_sph and E_elec_def_sph;
- Their (E_elec_int, E_elec_ext) coordinates wherein each of the two electrostatic energy densities are a linear combination, not necessarily the same, of E_elec_tot, E_elec_sph, E_elec_def, E_elec_def_sph, E_elec_nonuc, E_elec_nonuc_sph and/or E_elec_punctual.

10. The computer based method according to anyone of claims 2 to 9, wherein the analysis step (iv) can be followed by a step (v) of optimizing the interactions between the molecule and its environment by identifying unfavorable interactions, in particular in the (E_elec_int>0, E_elec_ext>0) or (F_int>0, F_ext>0); and/or (E_elec_int<0, E_elec_ext<0) or (F_int<0, F_ext<0) quadrants.

11. The computer based method according to anyone of claims 2 to 10, wherein the describing of step (iv) is performed by:

- fitting the simple linear regression line of said plurality of points, or of part of said plurality of points, and/or by computing the linear correlation coefficient R of said plurality of points, or of part of said plurality of points; or
- (a) fitting the simple linear regression line of said plurality of points, or of part of said plurality of points, and
(b) displaying a representation of said surface indicating for the plurality of points or part of it the deviation D from the regression line.

12. The computer based method according to anyone of claims 2 to 11, wherein the analysis step (iv) is followed by a step (v) of optimizing the interactions between the molecule and its environment by identifying the region(s) where the deviation from the regression line are the most important.

13. The computer based method according to anyone of preceding claims, for evaluating and comparing the stability of a plurality of structures, by computing, for each structure, the linear correlation coefficient R of said plurality of points, or of part of said plurality of points.

14. The computer based method according to claim 13, wherein the plurality of structures corresponds to:

- different structures or poses of a same molecule-environment couple;
- the molecular structures or poses of different molecule-environment complexes, the molecule or the environment being the same for all the complexes.

15. The computer based method according to anyone of preceding claims, for evaluating and comparing the stability of at least one structure of a plurality of complexes constituted of a molecule and its environment, the plurality of complexes corresponding in particular to:

- real or predicted crystal packings, the molecule being the same for all the complexes; or
- real or predicted co-crystals or crystals of salts, the molecule being the same for all the complexes.

## Figure 1

## Figure 2

## Figure 3

## Figure 4

**Figure 5**

E_def_ext                    guanine

y = -0.9278x - 0.001
R² = 0.901

E_def_int

**Figure 6**

E_def_ext_ straightened                    guanine

E_def_int_straightened

y = -0.9836x - 0.0003
R² = 0.9679

**Figure 7**

**Figure 8**

**Figure 9**

**Figure 10**

A

B

## Figure 11

## Figure 12

## Figure 13

## Figure 14

## Figure 15

pot_def_ext — metformin chloride — pot_def_int

## Figure 16

Metformin chloride — E_elec_def_ext

$y = -0.9779x + 0.0014$
$R^2 = 0.9726$

E_elec_def_int

**Figure 17**

**Figure 18**

Europäisches
Patentamt

European
Patent Office

Office européen
des brevets

# EUROPEAN SEARCH REPORT

Application Number

EP 21 30 6732

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AL-WAHAIBI LAMYA H ET AL: "Investigation of potential anti-malarial lead candidate 2-(4-fluorobenzylthio)-5-(5-bromothiophen-2-yl)-1,3,4-oxadiazole: Insights from crystal structure, DFT, QTAIM and hybrid QM/MM binding energy analysis", JOURNAL OF MOLECULAR STRUCTURE, ELSEVIER AMSTERDAM, NL, vol. 1175, 1 August 2018 (2018-08-01), pages 230-240, XP085504153, ISSN: 0022-2860, DOI: 10.1016/J.MOLSTRUC.2018.07.102 * abstract * * page 231, column 2, paragraph 4 * * page 232, column 2, paragraph 1 * ----- | 1-15 | INV. G16C20/30 G16B15/30 |
| X | SPACKMAN MARK A ET AL: "Electrostatic potentials mapped on Hirshfeld surfaces provide direct insight into intermolecular interactions in crystals", CRYSTENGCOMM, vol. 10, no. 4, 17 January 2008 (2008-01-17), pages 377-388, XP055925424, Retrieved from the Internet: URL:https://pubs.rsc.org/en/content/articl epdf/2008/ce/b715227b> * abstract * * page 382, column 1, paragraph 1 * ----- | 1-15 | |

TECHNICAL FIELDS SEARCHED (IPC)

G16C
G06F
G16B

The present search report has been drawn up for all claims

| Place of search | Date of completion of the search | Examiner |
|---|---|---|
| The Hague | 27 May 2022 | Nurmi, Jussi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................

& : member of the same patent family, corresponding document

EPO FORM 1503 03.82 (P04C01)

# EP 4 195 210 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **DOMAGALA, S. et al.** *Acta Crystallographica Section A: Foundations of Crystallography,* 2012, vol. 68 (3), 337-351
- **MOLČANOV, K. et al.** *Crystal Growth & Design,* 2018, vol. 19 (1), 391-402
- **EDINGTON, P. et al.** *Acta Crystallographica Section B,* 1974, vol. 30 (1), 204-206
- **DITTRICH, B. et al.** *Acta Crystallographica Section A: Foundations of Crystallography,* 2005, vol. 61 (3), 314-320
- **MEENASHI, R. ; SELVARAJU, K. ; STEPHEN, A. D. ; JELSCH, C.** *Journal of Molecular Structure,* 2020, vol. 1213, 128139
- **MEENASHI, R. et al.** *Journal of Molecular Structure,* 2020, vol. 1213, 128139
- **JELSCH, C. et al.** *Journal of Molecular Structure,* 2020, vol. 1205, 127600